# EUROPEAN PATENT APPLICATION

(11) **EP 2 711 374 A1**
(43) Date of publication of application: **26.03.2014**
(21) Application number: 12789433.5
(22) Date of filing: 18.05.2012
(51) Int. Cl.: C07K 14/79, C07K 1/02, C07K 1/34, A61K 38/16, A61P 27/02

(54) **NOVEL METALLOPROTEIN AND PROCESS FOR PRODUCING SAME, AND PROPHYLACTIC OR THERAPEUTIC AGENT FOR CORNEAL AND CONJUNCTIVAL DISEASES COMPRISING SAID METALLOPROTEIN**

(30) Priority: 20.05.2011 JP 2011113012; 20.05.2011 JP 2011113013
(71) Applicant: Keio University, Tokyo 108-8345 (JP); Kowa Company Ltd., Naka-ku Nagoya-shi Aichi 460-8625 (JP)
(72) Inventor: HIGUCHI, Akihiro, Tokyo 160-8582 (JP); INOUE, Hiroyoshi, Tokyo 160-8582 (JP); TSUBOTA, Kazuo, Tokyo 160-8582 (JP)
(74) Representative: Jacobi, Markus Alexander
(86) International application number: PCT/JP2012/062752
(87) International publication number: WO 2012/161112

(57) **Abstract**

The present invention provides: a novel metalloprotein, i.e., selenium-lactoferrin; and a process for producing the metalloprotein. Selenium-lactoferrin according to the present invention can be produced suitably by adding a selenium salt to a solution containing lactoferrin and/or apolactoferrin and then subjecting the resultant mixed solution to dialysis or ultrafiltration.

Selenium-lactoferrin according to the present invention has an excellent therapeutic effect on corneal and conjunctival diseases, and is suitable for mass production on an industrial scale.

## Description

The present invention relates to a novel metalloprotein and a process for producing it, and to a prophylactic or therapeutic agent for corneal and conjunctival diseases comprising the metalloprotein. More specifically, the invention relates to a protein comprising selenium and apolactoferrin wherein selenium is bonded to apolactoferrin, i.e. selenium-lactoferrin, and to a process for producing it, as well as to a prophylactic or therapeutic agent for corneal and conjunctival diseases comprising the metalloprotein. The selenium-lactoferrin of the invention is useful for prevention or treatment of corneal and conjunctival disease, such as dry eye, keratoconjunctivitis sicca, superficial punctate keratopathy, corneal erosion and corneal ulcer.

The cornea is a thin transparent tissue without blood vessels, having a thickness of about 1 mm, and having a fine, highly regular structure composed of the epithelial layer, Bowman's membrane, stromal layer, Descemet's membrane and endothelial cell layer. The cornea is a highly differentiated tissue that is located at the front surface of the eye ball and has transparency and refractive power for directing externally received light to photoreceptors on the retina, and because it is extremely important for visual perception while also being directly in contact with the external world, it functions as a barrier against chemical invasion and biological invasion from microorganisms and the like. A lacrimal layer is present on the epithelial layer of the cornea, and it keeps the eye in a constantly wetted state while preventing adhesion between the eyelid and conjunctiva, and maintaining a physiological state for the cornea and conjunctiva. Corneal epithelial cells have a cylindrical base but are flatter toward the surface, and epithelial cells that divide at the base gradually migrate upward and are eventually shed and carried off by lacrimal fluid. Corneal endothelial cells do not undergo cell division, and therefore are not regenerated even when shed. When keratoconjunctival damage caused by various corneal diseases such as dry eye, corneal ulcer, corneal epithelial detachment or keratitis is delayed in its repair or is prolonged and fails to undergo repair for some reason, this can impair the structure or function not only of the corneal epithelium but also of the stroma or endothelium, and can have serious effects on eyesight and the barrier function. Keratoconjunctival epithelial conditions such as repetitive corneal erosion or protracted corneal epithelial loss are such conditions. The repair process for keratoconjunctival epithelial damage involves covering of the sites of epithelial loss by migration of conjunctival epithelial cells and subsequent remodeling of the normal keratoconjunctiva by cellular division and differentiation, and factors involved in repair of the conjunctival epithelium have been reported (see NPL 1, for example).

Also, corneal damage such as superficial punctate keratopathy (SPK), characterized by multiple minute punctiform epithelial loss in the corneal epithelium, which is seen in dry eye associated with a number of conditions such as Sjogren's syndrome, Stevens-Johnson syndrome, Meibomian gland dysfunction and VDT (Visual Display Terminal) syndrome, dry eye or keratoconjunctivitis sicca following ocular surgery (cataract surgery, corneal transplantation or refraction surgery), and in drug-induced corneal epithelial impairment, neurotrophic keratitis, diabetic keratopathy and allergenic conjunctivitis, can also have serious effects on eyesight and barrier function (see NPL 2, for example). Corneal epithelium damage such as superficial punctate keratopathy (SPK) has also been a problem with contact lens wearers in the past (see NPL 3, for example).

Components such as fibronectin, EGF (Epidermal Growth Factor) and hyaluronic acid are used as treatment agents for corneal epithelial wounds. However, fibronectin is a blood preparation that requires purification using a special purification kit from the patient's own blood plasma, and because this is laborious and a burden on the patient, it is not widely used in the clinic. Also, while EGF has a fissiparous effect on corneal epithelial cells, it is almost never used in the clinic due to problems associated with inflammation, or with the side-effect of vascularization in diabetic keratopathy.

Hyaluronic acid is a glycosaminoglycan with a molecular weight of several hundred, with N-acetyl-D-glucosamine and D-glucuronic acid as the constituent sugars, and because of its water retaining effect it is used for dry eye patients. Hyaluronic acid also has an effect on adhesion and migration of corneal epithelial cells, but its proliferating effect on epithelial cells is weak. It is also problematic in that it tends to have higher viscosity at high concentrations.

In recent years, methods of treatment using selenoprotein P or its fragments have been proposed for corneal and conjunctival diseases, and these have been reported to have a curing effect on corneal epithelial impairment (see PTL 1). However, it is difficult to harvest large amounts of selenoprotein P since it resides in the serum, while it is also difficult to produce as a recombinant protein by genetic engineering methods, and it is therefore unsuitable for large-scale industrial production.

Lactoferrin, on the other hand, is an iron-binding glycoprotein present primarily in the lactation of mammals, and it is found in neutrophils, lacrimal fluid, saliva, nasal discharge, bile, semen and the like. Lactoferrin is an important protein for infant health maintenance and development, and in recent years it has been shown to have antimicrobial and antibacterial effects and is used in the food industry as well as various other fields. In the field of ophthalmology, in particular, there have been proposed the use of lactoferrin as a lacrimal component as an active ingredient in eye drops for treatment of dry eye, and the use of apolactoferrin, with the iron removed from lactoferrin, as an active ingredient in ophthalmologic agents and contact lens compositions (see PTLs 2 and 3).

Lactoferrin is an iron-binding glycoprotein with a molecular weight of approximately 80,000, and it has in its three-dimensional structure two iron-binding pockets each allowing binding of one iron atom. The bacteriostatic action of lactoferrin is thought to be as follows. Specifically, the empty iron-binding pockets in lactoferrin have chelating action on iron, and this activity depletes iron that is essential for the development of microorganisms, thus limiting their growth. Consequently, microorganisms that absolutely require iron during development are subject to the bacteriostatic action of lactoferrin. This bacteriostatic action of lactoferrin is seen particularly in the enteral environment. Thus, chelation by iron-binding pockets in the same protein is the focus of interest with regard to the bacteriostatic action of lactoferrin.

However, binding of the iron-binding pockets in lactoferrin with iron is generally about 2000 times compared to other cations, and since it has been considered to be specific for iron (NPL 4), no metalloproteins have been known that have other metals replacing iron in lactoferrin. In addition, this is poorly analogous to the effect of selenium-lactoferrin on eye diseases such as described by the present invention, and in fact no reports have been found on such an effect.

### [Citation List]

### [Patent literature]

[PTL 1] International Patent Publication No. WO2006/137426
[PTL 2] Japanese Patent Publication No. 4634809
[PTL 3] Japanese Unexamined Patent Application Publication No. 2007-137817

### Non-Patent Literature

[NPL 1] Suzuki K et al., Progress in Retinal and Eye Research., 22, 113-133 (2003)
[NPL 2] Ohashi, Y., "2. Superficial punctate keratopathy", Manabe, R, Kinoshita, S., Ohashi, Y., ed., "Corneal Clinical Science, 2nd Edition", p36-43(2003) Igaku Shoin.
[NPL 3] Hamano M., Japan Contact Lens Society Journal 37 1-6 (1995)
[NPL 4] Lonnerdal B et al., Am J Clin Nutr, 41, 550-559 (1985)

### Summary of Invention

### Technical Problem

Thus, various treatment agents for corneal and conjunctival disease have been known in the prior art, but none have been known that have a high therapeutic effect and are suitable for large-scale industrial production, and therefore a demand exists for treatment agents that are superior in this regard. More excellent treatment agents are also desired for ophthalmologic conditions such as dry eye, keratoconjunctivitis sicca, superficial punctate keratopathy, corneal erosion and corneal ulcer.

### Solution to Problem

As a result of much diligent research in light of these problems, the present inventors have found that a metalloprotein having selenium bonded to apolactoferrin exhibits an excellent curative effect on keratoconjunctival damage, and the invention has been completed.

Specifically, the invention provides the following (1) to (11).
(1) A metalloprotein that is selenium-lactoferrin.
(2) The metalloprotein of (1), wherein selenium is bonded to an iron-binding pocket in apolactoferrin.
(3) A process for producing the metalloprotein according to (1) or (2) above, the process comprising a step of adding a selenium salt to a solution containing lactoferrin and/or apolactoferrin, and a step of subjecting the obtained mixed solution to dialysis or ultrafiltration.
(4) The process of (3), employing a solution that contains apolactoferrin.
(5) A process for producing a metalloprotein according to (4) above, wherein apolactoferrin is produced by a process comprising a step of supplying a solution containing lactoferrin and an acid to an ultrafiltration membrane and conducting ultrafiltration, and a step of recovering the solution that has not permeated the ultrafiltration membrane.
(6) A metalloprotein produced by the process according to any one of (3) to (5) above.
(7) A prophylactic or therapeutic agent for corneal and conjunctival diseases, comprising a metalloprotein according to (1), (2) or (6) above as an active ingredient.
(8) The prophylactic or therapeutic agent of (7), wherein the corneal or conjunctival disease is dry eye, keratoconjunctivitis sicca, superficial punctate keratopathy, corneal erosion or corneal ulcer.
(9) A prophylactic or therapeutic agent for dry eye, keratoconjunctivitis sicca, superficial punctate keratopathy, corneal erosion or corneal ulcer, comprising a metalloprotein according to (1), (2) or (6) above as an active ingredient.
(10) The prophylactic or therapeutic agent according to any one of (7) to (9), which is an eye drop or eye ointment.
(11) An ophthalmologic composition comprising a metalloprotein according to (1), (2) or (6) above, and an ophthalmological carrier acceptable as an ophthalmological formulation.

The invention further provides the following (12) to (15).
(12) The use of a metalloprotein according to (1), (2) or (6) above as an active ingredient in a prophylactic or therapeutic agent for corneal and conjunctival diseases.
(13) The use of a metalloprotein according to (1), (2) or (6) above for production of a prophylactic or therapeutic agent for corneal and conjunctival diseases.
(14) A metalloprotein according to (1), (2) or (6) above, which is to be used for prevention or treatment of a corneal or conjunctival disease.
(15) A method for prevention or treatment of a corneal or conjunctival disease such as dry eye, keratoconjunctivitis sicca, superficial punctate keratopathy, corneal erosion or corneal ulcer, and preferably a dry eye, keratoconjunctivitis sicca, superficial punctate keratopathy, corneal erosion or corneal ulcer accompanied by keratoconjunctival epithelial damage, the method comprising administration of an effective dose of selenium-lactoferrin to a patient suffering from a corneal or conjunctival disease such as dry eye, keratoconjunctivitis sicca, superficial punctate keratopathy, corneal erosion or corneal ulcer, and preferably dry eye, keratoconjunctivitis sicca, superficial punctate keratopathy, corneal erosion or corneal ulcer accompanied by conjunctival epithelial damage, or a patient suspected of suffering from such a disease.

### Advantageous Effects of Invention

Lactoferrin is abundantly present in mammalian body fluids such as milk, while also being obtainable by genetic engineering methods, and it is therefore readily available in large amounts. Therefore, the selenium-lactoferrin of the invention obtained using this as starting material can also be produced at low cost on a large industrial scale. In addition, the selenium-lactoferrin of the invention has a high therapeutic effect for corneal and conjunctival disease. Furthermore, since selenium-lactoferrin has antimicrobial activity and may make it possible to reduce the amounts of antiseptic agents in formulations or to completely avoid the use of antiseptic agents,it can alleviate side-effects associated with antiseptic agents. That is, the present invention can provide a therapeutic agent for corneal and conjunctival diseases that has a high therapeutic effect and high safety, and is suitable for large-scale industrial production.

### Brief Description of Drawings

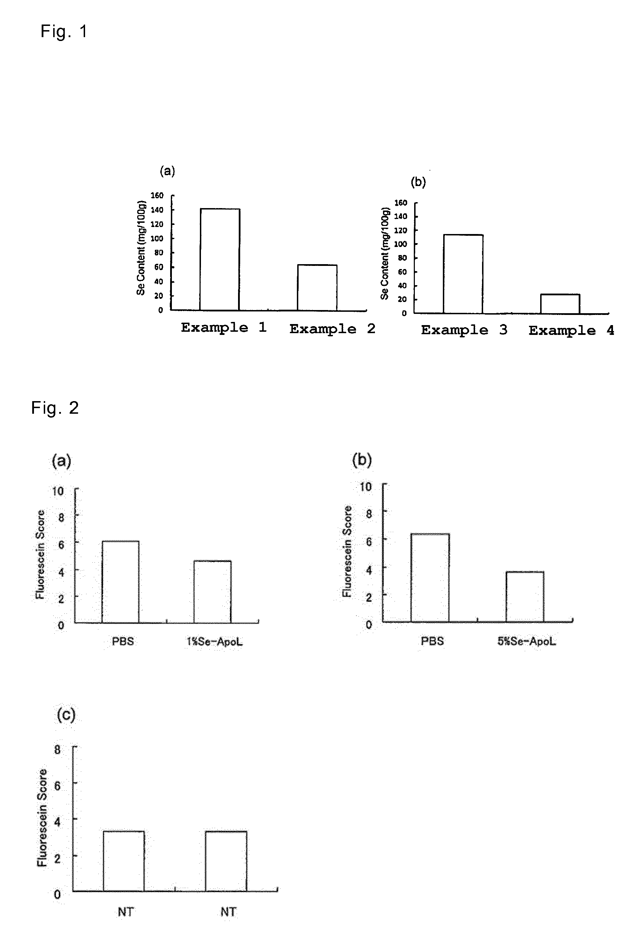

Fig. 1 shows the results of comparing amounts of selenium in selenium-lactoferrin of the invention, with production using apolactoferrin as starting material and using lactoferrin as starting material. Fig. 1(a) shows the results of using a dialysis membrane to remove excess selenium that has not been taken up into protein, and (b) shows the results of using an UF membrane.
Fig. 2 shows results for the degree of preventing corneal epithelial damage by the selenium-lactoferrin of the invention using an orbital lacrimal gland-extracted dry eye model, in comparison to phosphate-buffered saline (PBS). Fig. 2(a) shows comparison with 1% selenium-lactoferrin, (b) shows comparison with 5% selenium-lactoferrin and (c) shows a normal group. In the graphs, the ordinate represents the fluorescein score value, the left column shows the results for the left eye administered PBS (control), and the right column (Fig. 2(a) and (b)) shows the results for the right eye administered selenium-lactoferrin.

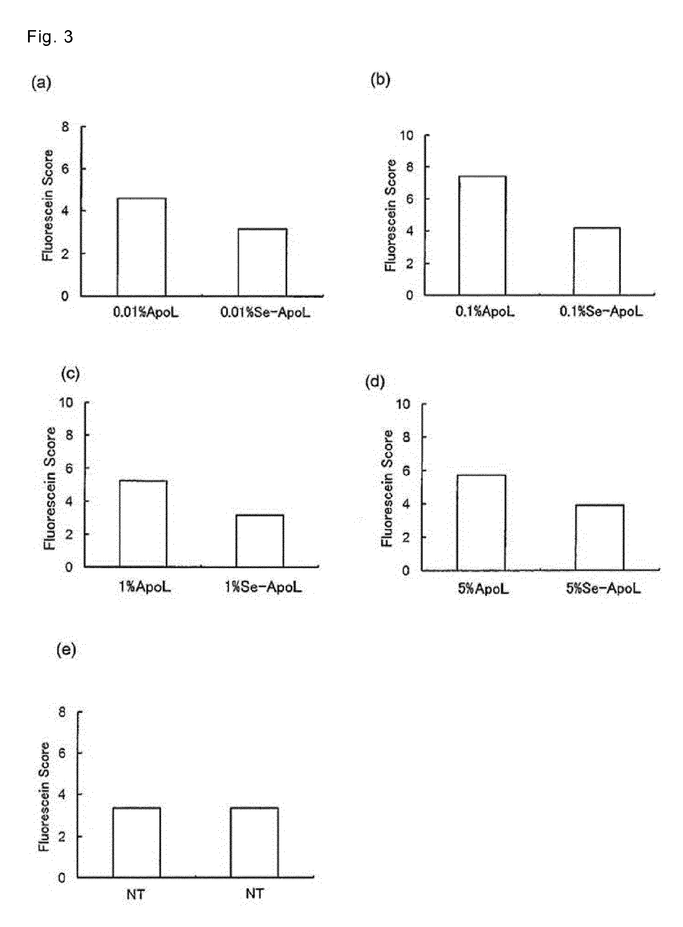

Fig. 3 shows results for the degree of preventing corneal epithelial damage by the selenium-lactoferrin of the invention using an orbital lacrimal gland-extracted dry eye model, in comparison to an equal concentration of apolactoferrin. Fig. 3(a) to (d) show comparisons of 0.01%, 0.1%, 1% and 5% concentrations of selenium-lactoferrin with equal concentrations of apolactoferrin, and (e) shows a normal group. In the graphs, the ordinate represents the fluorescein score value. The left columns in (a) to (d) show the results for the left eye administered apolactoferrin, and the right column shows the results for the right eye administered selenium-lactoferrin. Both columns in (e) shows the results for the left and right eyes administered PBS.

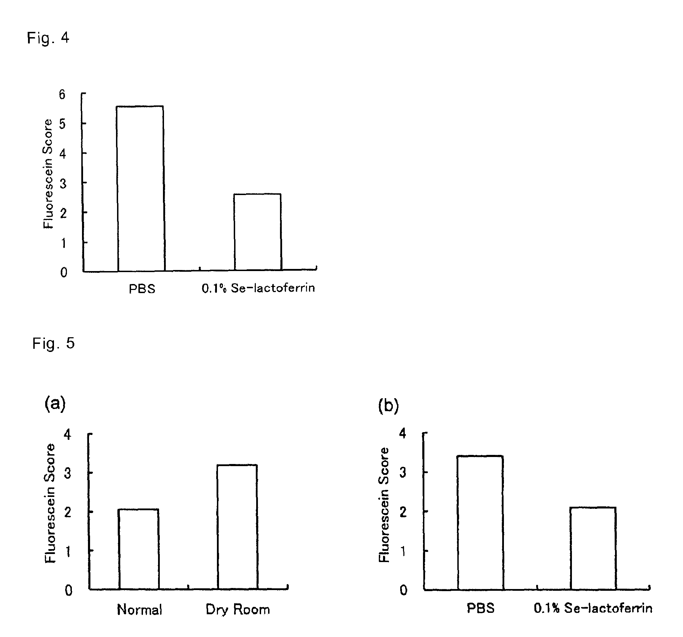

Fig. 4 shows results for the degree of preventing corneal epithelial damage by the selenium-lactoferrin of the invention using a smoke-treated dry eye model, in comparison to PBS. In the graph, the ordinate represents the average fluorescein score value, the left column shows the side administered PBS as a control, and the right column shows side administered 0.1% selenium-lactoferrin.
Fig. 5 shows results for the degree of preventing corneal epithelial damage by the selenium-lactoferrin of the invention using a dry room-treated dry eye model, in comparison to PBS. In the graphs, the ordinate represents the average fluorescein score value. The left column of Fig. 5(a) shows the score for a normal environment, and the right column shows the score for dry room treatment (PBS-administered side). The left column of Fig. 5(b) represents the PBS-administered side as a control, and the right column represents the 0.1% selenium-lactoferrin-administered side.

### Description of Embodiments

The term "selenium-lactoferrin" used according to the invention refers to a metalloprotein comprising selenium and apolactoferrin wherein selenium is bonded to apolactoferrin. As explained in the examples below, the selenium in the selenium-lactoferrin of the invention is not removed by dialysis or ultrafiltration, but is only removed by rendering the pH of the solution strongly acidic, and therefore the selenium is not present in the form of a simple mixture in apolactoferrin but rather is bound to apolactoferrin with a given strength. Thus, the selenium-lactoferrin of the invention is different from a simple mixture of selenium and apolactoferrin. Incidentally, it is believed that the selenium and apolactoferrin in the selenium-lactoferrin of the invention are in a relationship with the selenium bound by the chelating effect of the iron-binding pocket in apolactoferrin, but it is not our intention to restrict the invention to this theory.

The term "apolactoferrin" used according to the invention refers to lactoferrin with the iron removed. The apolactoferrin of the invention may be derived from natural lactoferrin obtained from animal body fluid such as milk, synthesized by genetic engineering, having some of the amino acids replaced by other amino acids, having some of the amino acids deleted, or having several amino acids added.

The process for producing selenium-lactoferrin according to the invention will now be explained in detail. The selenium-lactoferrin of the invention can be produced by using a solution containing lactoferrin and/or apolactoferrin as the starting material and adding a selenium salt thereto to bind selenium to the apolactoferrin, and then removing the excess selenium by dialysis or ultrafiltration of the solution. Without being restricted to any particular theory, it is believed that the selenium-lactoferrin of the invention is formed when selenium binds to an empty iron-binding pocket in lactoferrin or apolactoferrin. It is also believed to be formed when selenium binds to the iron-binding pocket in lactoferrin by substituting for iron that is bound in the pocket. Therefore, the starting material used may be a solution containing either or both lactoferrin and apolactoferrin. In the production process of the invention, binding of selenium to an empty iron-binding pocket in lactoferrin or apolactoferrin is thought to occur more readily than substitution for iron in lactoferrin, and therefore from the viewpoint of production efficiency it is preferred to use a solution containing apolactoferrin as the starting material. In addition, when a solution containing both lactoferrin and apolactoferrin is to be used, a higher apolactoferrin content is preferred, and for example, the apolactoferrin content is 90 mol% or greater, especially 95 mol% or greater and most preferably 97 mol% or greater with respect to the total amount of lactoferrin and apolactoferrin.

The solution containing the lactoferrin and/or apolactoferrin may be prepared using a commercial product available as lactoferrin or apolactoferrin as a pharmaceutical or reagent. The solution containing lactoferrin and/or apolactoferrin may be prepared from an animal-derived substance obtained from milk (for example, cow milk) or whey, or it may be prepared from a product produced by a genetic engineering method. Also, there may be used a product produced by increasing the apolactoferrin concentration of a solution containing lactoferrin and apolactoferrin using any of various methods known in the prior art, for example, adding an acid such as hydrochloric acid or citric acid to a lactoferrin-containing solution extracted from whey or the like to adjust the pH to about 2 and promote dissociation of iron. A solution with increased apolactoferrin concentration can also be suitably produced, for example, by the method described in Japanese Patent Publication No. 4634809, namely mixing a lactoferrin-containing solution with an acid, supplying the liquid mixture to an ultrafiltration membrane, removing the non-permeating solution and the permeating solution separately out of the system, re-adding an acid to the non-permeating solution, conducting further ultrafiltration, and repeating this procedure until the target protein concentration and separation rate are reached. In this case, the acid used may be an inorganic acid such as hydrochloric acid, sulfuric acid, nitric acid, phosphoric acid or carbonic acid or an organic acid such as acetic acid, benzoic acid or citric acid. The ultrafiltration may be carried out using, for example, an ultrafiltration apparatus which is a pencil-type module small laboratory apparatus (Model PS-24001) by Asahi Kasei Chemicals Corp. incorporating an ACP-0013 UF module by the same manufacturer (hollow fiber module: membrane inner diameter = 0.8 mm, effective membrane area = 170 cm², membrane material = polyacrylonitrile, nominal molecular cutoff = 13,000).

The selenium salt to be used in the production process of the invention may be of any type and is not particularly restricted so long as it is water-soluble, and selenium chloride or selenium bromide may be used, for example. The amount of selenium salt added is not particularly restricted, but since selenium is thought to bind in the iron-binding pocket of lactoferrin or apolactoferrin and two iron-binding pockets are present per molecule of the protein, it is preferred to add the selenium salt to the reaction system in an amount so that approximately 2 mol or more of selenium is present to 1 mol of protein. Also, since lactoferrin or apolactoferrin becomes denatured in an excessively high pH range and selenium fails to bind in an excessively low pH range, the pH of the reaction mixture is preferably between 3 and 9. The pH of the reaction mixture can be adjusted by using any of various known buffering agents. The reaction temperature and time for the protein and selenium salt may be appropriately adjusted depending on the material and amount thereof used, but generally mixing at 10°C to 35°C for about 5 minutes to 2 hours is preferred.

After mixing the lactoferrin or apolactoferrin with the selenium salt, the excess selenium that has not been bound with protein has cytotoxicity and must be removed, and this excess selenium can be removed by dialysis or ultrafiltration. When the excess selenium is to be removed by dialysis, a dialysis membrane is used for 6 hours to 72 hours of dialysis in a 10- to 10,000-fold amount of water. Also, when the excess selenium is to be removed by ultrafiltration, there may be used a common commercially available apparatus such as one having a structure comprising a separation fluid tank, a fluid-supply pump and an ultrafiltration module with an ultrafiltration membrane. The dialysis membrane and ultrafiltration membrane to be used in the production process of the invention is preferably one with a molecular cutoff of about 5,000 to about 80,000, since lactoferrin or apolactoferrin has a molecular weight of about 80,000. The material of the dialysis membrane may be regenerated cellulose, cellulose acetate, polyacrylonitrile or the like, and the material of the ultrafiltration membrane may be, for example, an organic film of a natural or synthetic polymer such as cellulose acetate, polysulfone, polyethersulfone, polyacrylamide, polyimide, aromatic polyamide, polyacrylonitrile or hydrophilic polyolefin, or a ceramic inorganic membrane of alumina, zirconia, titanium and the like. Also, the form of the ultrafiltration membrane may be a hollow fiber module type, flat plate module type or flat membrane type, but from the viewpoint of the filtration rate it is preferred to select a hollow fiber module type. The ultrafiltration is also preferably carried out at 20°C or higher from the viewpoint of filtration efficiency.

The selenium-lactoferrin of the invention has an excellent curative effect in a rat corneal epithelial damage model, as demonstrated by the experimental examples described below. Thus, drugs containing the same are useful as prophylactic or therapeutic agents for diseases associated with inflammation or loss of the cornea or conjunctiva, such as corneal and conjunctival disease, more specifically, dry eye, keratoconjunctivitis sicca, superficial punctate keratopathy, corneal erosion and corneal ulcer, and especially as prophylactic or therapeutic agents for such conditions accompanied by keratoconjunctival epithelial damage.

The term "keratoconjunctiva" used in the present specification means the cornea and/or conjunctiva, and "corneal and conjunctival disease" is a disease of the cornea and/or conjunctiva. Diseases such as dry eye, keratoconjunctivitis sicca, superficial punctate keratopathy, corneal erosion and corneal ulcer according to the invention are generally included among corneal and conjunctival diseases, but they do not necessarily have to be caused by corneal and conjunctival diseases. Thus, dry eye, keratoconjunctivitis sicca, superficial punctate keratopathy, corneal erosion and corneal ulcer according to the invention also include such conditions not associated with corneal and conjunctival disease.

The phrase "pharmaceutically acceptable carrier" as it pertains to the invention means a pharmaceutical carrier that is acceptable for production of oral formulations such as tablets, capsules and liquid drugs, and the phrase "ophthalmological carrier that is acceptable for ophthalmological formulations" means a pharmaceutical carrier acceptable for production of ophthalmological formulations such as eye drops or eye ointments.

A medical composition containing the selenium-lactoferrin of the invention as an active ingredient may further contain a pharmaceutically acceptable carrier, or an active ingredient other than selenium-lactoferrin. The selenium-lactoferrin of the invention may be administered directly to the eyes as an ophthalmological formulation, or it may be used as a contact lens storage solution. The selenium-lactoferrin of the invention may also be used as an oral formulation.

Oral formulations include solid oral formulations such as tablets, capsules, granules, film coating agents and powders, and liquid oral formulations such as liquid drugs and syrups.

For preparation of a solid oral formulation, an excipient, and if necessary a binder, disintegrator, lubricant, coloring agent, taste corrective, odor corrective or the like, may be added, and then tablets, granules, powders, capsules or the like may be prepared by a common method. Such additives may be ones that are commonly used in the relevant field, and for example, excipients include lactose, sodium chloride, glucose, starch, microcrystalline cellulose and silicic acid; binders include water, ethanol, propanol, simple syrup, gelatin solution, hydroxypropyl cellulose, methyl cellulose, ethyl cellulose, shellac, calcium phosphate and polyvinylpyrrolidone; disintegrators include agar powder, sodium hydrogencarbonate, sodium lauryl sulfate and stearic acid monoglyceride; lubricants include purified talc, stearic acid salts, borax and polyethylene glycol; coloring agents include β-carotene, yellow iron sesquioxide and caramel; and taste correctives include saccharose and bitter orange.

For preparation of a liquid oral formulation, a taste corrective, buffering agent, stabilizer, preservative or the like may be added to produce an internal liquid drug, syrup or elixir. Such additives may be ones commonly used in the relevant field, and for example, taste correctives include saccharose; buffering agents include sodium citrate; stabilizers include tragacanth; and preservatives include paraoxybenzoic acid esters.

A medical composition containing the selenium-lactoferrin of the invention as an active ingredient is preferably an ophthalmological formulation and preferably an eye drop formulation, and such eye drops may be aqueous eye drops, nonaqueous eye drops, suspended eye drops, emulsified eye drops or an eye ointment. Such a formulation can be produced as a composition suitable for administration by a method known to those skilled in the art, with addition of a pharmaceutically acceptable carrier if necessary, with an isotonizing agent, chelating agent, stabilizer, pH regulator, antiseptic agent, antioxidant, dissolving aid, thickening agent or the like.

Isotonizing agents include saccharides such as glucose, trehalose, lactose, fructose, mannitol, xylitol and sorbitol, polyhydric alcohols such as glycerin, polyethylene glycol and propylene glycol and inorganic salts such as sodium chloride, potassium chloride and calcium chloride, with contents of preferably 0 to 5 wt% of the entire composition.

Chelating agents include edetic acid salts such as disodium edetate, calcium edetate disodium, trisodium edetate, tetrasodium edetate and calcium edetate, ethylenediaminetetraacetic acid salts, nitrilotriacetic acid and its salts, sodium hexametaphosphate, citric acid and the like, with contents of preferably 0 to 0.2 wt% of the entire composition.

Stabilizers include sodium hydrogen sulfite, in a content of preferably 0 to 1 wt% of the entire composition.

Various pH regulators include acids such as hydrochloric acid, carbonic acid, acetic acid and citric acid, and bases including alkali metal hydroxides such as sodium hydroxide and potassium hydroxide, alkali metal carbonates or hydrogencarbonates such as sodium carbonate, alkali metal acetic acid salts such as sodium acetate, alkali metal citric acid salts such as sodium citrate, and trometamol, with contents of preferably 0 to 20 wt% of the entire composition.

Although the selenium-lactoferrin of the invention itself can exhibit antimicrobial activity, when an antiseptic agent is necessary it may be a paraoxybenzoic acid ester such as sorbic acid, potassium sorbate, methyl paraoxybenzoate, ethyl paraoxybenzoate, propyl paraoxybenzoate or butyl paraoxybenzoate, a quaternary ammonium salt such as chlorhexidine gluconate, benzalkonium chloride, benzethonium chloride or cetylpyridinium chloride, an alkylpolyaminoethylglycine, chlorobutanol, Polyquad, polyhexamethylene biguanide, chlorhexidine or the like, with a content of preferably 0 to 0.2 wt% of the entire composition.

Antioxidants include sodium hydrogen sulfite, dry sodium sulfite, sodium pyrosulfite and concentrated mixed tocopherols, in contents of preferably 0 to 0.4 wt% of the entire composition.

Dissolving aids include sodium benzoate, glycerin, D-sorbitol, glucose, propylene glycol, hydroxypropyl methyl cellulose, polyvinylpyrrolidone, macrogol and D-mannitol, in contents of preferably 0 to 3 wt% of the entire composition.

Thickening agents include polyethylene glycol, methyl cellulose, ethyl cellulose, carmellose sodium, xanthan gum, chondroitin sulfate sodium, hydroxyethyl cellulose, hydroxypropyl cellulose, hydroxypropyl methyl cellulose, polyvinylpyrrolidone and polyvinyl alcohol, in contents of preferably 0 to 70 wt% of the entire composition.

When an eye drop is to be prepared, this may be accomplished by, for example, dissolving or suspending the desired components mentioned above in an aqueous solvent such as sterilized distilled water or physiological saline or a nonaqueous solvent which may be a vegetable oil such as cottonseed oil, soybean oil, sesame oil, peanut oil or the like, adjusted to the prescribed osmotic pressure, and subjected to sterilization treatment such as mechanical sterilization.

When an eye ointment is to be prepared it may contain an ointment base in addition to the various components mentioned above. There are no particular restrictions on such an ointment base, but it is preferably an oil base such as vaseline, liquid paraffin or polyethylene; an emulsion base prepared by emulsification of an oil phase and aqueous phase with a surfactant or the like; or a water-soluble base comprising hydroxypropyl methyl cellulose, carboxymethyl cellulose, polyethylene glycol or the like.

When a prophylactic or therapeutic agent for corneal and conjunctival diseases comprising the selenium-lactoferrin of the invention as an active ingredient is to be administered, the dosage will differ depending on the body weight, age, gender and symptoms of the patient and the form of administration and frequency of administration, but it will usually be 0.2 to 1000 µg and preferably 2 to 200 µg per day as selenium-lactoferrin for an adult, administered either all at once or in divided doses, or for a liquid eye drop it may be 0.01 to 50 mg/mL and preferably 0.1 to 10 mg/mL, several times per day with one to several drops each time.

The present invention will now be further explained by examples, with the understanding that the invention is not limited in any way by the examples.

### Examples

### [Example 1]

### Preparation of selenium-lactoferrin

After adding 1 g of apolactoferrin (product of Upwell Co., Ltd.) to an aqueous solution containing 5.4 mg of selenium(I) chloride (product of Wako Pure Chemical Industries, Ltd.), the liquid volume was adjusted to 10 mL, the aqueous solution pH was confirmed to be 3 or higher, and then a stirrer was used for stirring 240 times/min at 25°C for a period of 30 minutes, to bind selenium to the apolactoferrin. Next, the mixed solution of selenium(I) chloride and apolactoferrin was transferred to a cellulose tube for dialysis (molecular cutoff: 12,000 to 14,000; product of As One Corp.), and 6 L of distilled water (≥17 MΩ) was used for dialysis at 4°C for 48 hours while exchanging the distilled water every 12 hours, and the excess selenium not bound to apolactoferrin was removed. Following dialysis, freeze-drying was carried out using an FDU-12AS Freeze Dryer (product of As One Corp.).

### [Example 2]

The steps of Example 1 were repeated, except that lactoferrin (product of Tatua Japan) was used as the starting material.

### [Example 3]

The steps of Example 1 were repeated, but for this example the excess selenium was removed by the following procedure instead of dialysis. Specifically, for this example a UF membrane (Microza AP-0013(UF), molecular cutoff: 6,000, product of Asahi Kasei Corp.) was set in a pencil-type module benchtop filtration apparatus (Microza UF·M FPS-24001, product of Asahi Kasei Corp.). Also, the apparatus (operating pressure: 40 kPa as the module outlet pressure) was used for ultrafiltration of 10 mL of the mixed solution of selenium(I) chloride and apolactoferrin three times at room temperature (≥20°C).

### [Example 4]

The steps of Example 3 were repeated, except that lactoferrin (product of Tatua Japan) was used as the starting material.

### [Example 5]

### Measurement of selenium binding

### (Experiment method)

After placing 1 g of the freeze-dried selenium-lactoferrin produced in Examples 1 to 4 in a Kjeldahl flask, 10 mL of nitric acid (product of Wako Pure Chemical Industries, Ltd.) was added and the mixture was allowed to stand at room temperature for 4 hours. It was then gently heated for 15 minutes with a direct flame, and further heated for 10 minutes with a moderately strong flame. After natural cooling, 5 mL of 70% perchloric acid (product of Wako Pure Chemical Industries, Ltd.) was added and the mixture was concentrated by heating. After the white smoke of perchloric acid was generated, heating was continued for another 15 minutes. The decomposition product had a faint yellow color. After natural cooling, 1 mL of water was added, the mixture was concentrated by heating, and upon confirming white smoke, heating was continued for another 2 minutes. After natural cooling, the same procedure was repeated. Next, 1 mL of 10% hydrochloric acid (product of Wako Pure Chemical Industries, Ltd.) was added and heating was performed for 30 minutes in a boiling water bath. This was diluted with distilled water to a total volume of 5 mL. The diluted solution was used as the test solution.

Upon sampling 2 mL each for a calibration curve solution and a test solution, 4 mL of a 0.1 mol/L EDTA (product of Wako Pure Chemical Industries, Ltd.) solution and 2 mL of a 20% hydroxylamine hydrochloride (product of Wako Pure Chemical Industries, Ltd.) solution were added, and 10% hydrochloric acid (product of Wako Pure Chemical Industries, Ltd.) and 10% ammonia water (product of Wako Pure Chemical Industries, Ltd.) were used to adjust the pH to between 1.0 and 1.5. Next, 5 mL of a 0.1% 2,3-diaminonaphthalene (product of Wako Pure Chemical Industries, Ltd.) solution was added and mixed therewith. The cover was closed, and the mixture was warmed in dark surroundings for 30 minutes in a water bath at 50°C. After natural cooling, the entire liquid was transferred to a separatory funnel, 10 mL of cyclohexane (product of Wako Pure Chemical Industries, Ltd.) was added, and after shaking for 5 minutes, the cyclohexane layer was separated off. The cyclohexane solution was rinsed twice with 25 mL of 0.1 mol/L hydrochloric acid (product of Wako Pure Chemical Industries, Ltd.). The cyclohexane alone was removed out, and a fluorescence spectrophotometer (FP6000, product of JASCO Corp.) was used to measure the fluorescence intensity at an excitation wavelength of 378 nm and a fluorescent wavelength of 520 nm. The selenium concentration of the test solution was measured from the value on the calibration curve.

### (Results)

The results are shown in Fig. 1. In Fig. 1, (a) shows the results after removing excess selenium using a dialysis membrane, and (b) shows the results after removing excess selenium by ultrafiltration using an UF membrane. In the graphs, the ordinate represents the amount of selenium (mg) bound to 100 g of apolactoferrin, the left column representing the selenium binding when apolactoferrin was used as starting material and the right column representing the selenium binding when lactoferrin was used as starting material.

In the left columns of (a) and (b) when apolactoferrin was used as starting material, the selenium binding values were 142 mg/100 g and 114 mg/100 g, respectively, whereas in the right columns of (a) and (b) when lactoferrin was used as starting material, the selenium binding values were 64 mg/100 g and 28 mg/100 g, respectively. These results confirmed that production efficiency for selenium-lactoferrin as the final product was higher when using apolactoferrin than when using lactoferrin as the starting material.

### [Experimental Example 1]

### Therapeutic efficacy test for keratoconjunctival epithelial damage

A dry-eye keratoconjunctival epithelial damage model was prepared by the method described below, based on the method of Fujiwara et al. (Invest. Ophthalmol. Vis. Sci. 42, 96-100, 2001), and the selenium-lactoferrin produced in Example 3 was used to evaluate the curative effect on keratoconjunctival epithelial damage. The experiment was conducted using both eyes of each individual, with one eye as the control and administering the drug solution to the other eye, to allow comparison in the same individual.

### (Experiment method)

After intraperitoneally administering pentobarbital (35 mg/kg) to male SD rats (7-weeks-old) and subjecting them to general anesthesia, the orbital lacrimal glands on both sides were extracted to prepare a dry eye model.

On the day following extraction of the lacrimal gland, PBS was dropped into the left eye, while PBS (Control group), 1% selenium-lactoferrin (PBS solution) or 5% selenium-lactoferrin (PBS solution) was dropped into the right eye, in each group for 2 consecutive weeks, 4 times a day at 5 µL each time. Animals not subjected to lacrimal gland extraction were used as a normal group, and PBS was dropped into both eyes under the same conditions (normal group). Nine or ten animals were used in each group.

Upon completing 3 weeks of eye dropping, the damaged portions of the corneal epithelia were dyed with the fluorescent dye fluorescein.

The degree of corneal epithelial damage was determined by scoring damage on the following scale for each of nine sections defined by dividing the total cornea into top, middle, bottom and left, center, right, and calculating the total value. Next, the total score value for the left eye and the total score value for the right eye were compared by Student's t-test, for each group. For unbiased evaluation, the contents of the drug solution administered to each group were blinded from the start of dropping until scoring of corneal epithelial damage, and matched after scoring.

### (Corneal epithelium fluorescein dye score judgment scale)

0: Not dyed (no punctiform fluorescence)
1: Slight punctiform fluorescence seen
2: Relatively large amount of punctiform fluorescence seen
3: Dense punctiform fluorescence seen

### (Results)

The results are shown in Fig. 2. In Fig. 2, (a) shows the 1% selenium-lactoferrin (PBS solution)-administered group (hereunder, "S1 group"), (b) shows the 5% selenium-lactoferrin (PBS solution)-administered group (hereunder, "S5 group"), and (c) shows the normal group (hereunder, "N group"). In the graph, the ordinate represents the fluorescein score value, the left column shows the left eye, i.e. the PBS-administered side of the control, and the right column shows the right eye, i.e. the side administered selenium-lactoferrin (denoted as Se-ApoL in the graph; (a), (b)) or PBS (c). Each fluorescein score value was expressed as an average value.

In the left columns of (a) and (b), the average fluorescein score is 6.1 to 6.3, clearly showing that corneal epithelial damage had progressed by 2 weeks of PBS administration following lacrimal gland extraction. In the N group of (c) without lacrimal gland extraction, the average fluorescein scores were 3.3 (left eye) and 3.3 (right eye) by 2 weeks of PBS administration.

On the other hand, as shown by the right columns of (a) and (b), the fluorescein score averages were 4.7 in the S1 group of (a) and 3.7 in the S5 group of (b), confirming that progression of corneal epithelial damage had been inhibited compared to the left eyes.

### [Experimental Example 2]

The pharmacological test of Experimental Example 1 was repeated, but four drug-administered groups were formed in addition to the normal group, with administration of selenium-lactoferrin at a concentration of 0.01%, 0.1%, 1% or 5% to the right eye and apolactoferrin at the same concentration to the left eye.

### (Results)

The results are shown in Fig. 3. In Fig. 3, (a) shows the 0.01% selenium-lactoferrin (PBS solution)-administered group (hereunder, "S0.01 group"), (b) shows the 0.1% selenium-lactoferrin (PBS solution)-administered group (hereunder, "S0.1 group"), (c) shows the 1% selenium-lactoferrin (PBS solution)-administered group (hereunder, "S1 group"), (d) shows the 5% selenium-lactoferrin (PBS-solution)-administered group (hereunder, "S5 group") and (e) shows the normal group (hereunder, "N-group"). In the graph, the ordinate represents the fluorescein score value, the left column shows the left eye, i.e. the side administered apolactoferrin (denoted as ApoL in the graph; (a) to (d)) or PBS (e), and the right column shows the right eye, i.e. the side administered selenium-lactoferrin (denoted as Se-ApoL in the graph; (a) to (d)) or PBS (e). Each fluorescein score value was expressed as an average value.

In the left column of (a), the average fluorescein score was 4.6, clearly showing that corneal epithelial damage had progressed. On the other hand, as shown by the right column of (a), the fluorescein score average was 3.2, confirming that progression of corneal epithelial damage had been inhibited compared to the left eye. The same trend was also found in (b) to (d).

### [Experimental Example 3]

### Therapeutic efficacy test in smoker rats

A dry-eye keratoconjunctival epithelial damage model was prepared by the method described below, based on the method of Higuchi et al. (Higuchi A, Ito K, Dogru M, Kitamura M, Mitani F, Kawakita T, Ogawa Y, Tsubota K., Corneal damage and lacrimal glands dysfunction in a smoking rat model. Free Radic Biol Med., 2011; 51:2210-16), and the selenium-lactoferrin produced in Example 3 was used to evaluate the curative effect on keratoconjunctival epithelial damage. The experiment was conducted using both eyes of each individual, with one eye as the control and administering the drug solution to the other eye, to allow comparison in the same individual.

### (Experiment method)

Male SD rats (7 weeks old) were subjected to smoking treatment to produce a dry eye model.

Specifically, mainstream smoke (300 mL) was added to the rat-containing chamber six times at 30 minute intervals for 5 days of treatment, to elicit keratoconjunctival epithelial damage. The eye drop conditions were PBS in one eye and 0.1% selenium-lactoferrin (PBS solution) in the other eye, at 5 µL each time, once before smoking treatment and 3 afterwards for a total of 4 times per day, for 5 consecutive days. Nine animals were used in each group.

Upon completion of 5 days of smoking treatment and dropping administration, the corneal epithelial damaged portions were evaluated in the same manner as Example 1, by dyeing with the fluorescent dye fluorescein and scoring. The total score value for the PBS-administered side and the total score value for 0.1% selenium-lactoferrin were compared by a Student's t-test.

### (Results)

The results are shown in Fig. 4. In the graphs, the ordinate represents the average fluorescein score value. The left column represents the PBS-administered side as a control, and the right column represents the 0.1% selenium-lactoferrin-administered side.

As shown in Fig. 4, the keratoconjunctival epithelial damage caused by 5 days of smoking treatment was clearly inhibited to a significant degree (p <0.001) by administration of 0.1% selenium-lactoferrin (fluorescein score average: 2.6), compared to the control (PBS-administered side, fluorescein score average: 5.6).

### [Experimental Example 4]

### Therapeutic efficacy test on dry room mice

A dry-eye keratoconjunctival epithelial damage model was prepared by the following method, and the selenium-lactoferrin produced in Example 3 was used to evaluate the curative effect on keratoconjunctival epithelial damage. The experiment was conducted using both eyes of each individual, with one eye as the control and administering the drug solution to the other eye, to allow comparison in the same individual.

### (Experiment method)

Male ICR mice (6 weeks old) were treated in a dry room to prepare a dry eye model.

Specifically, the mice were treated for 2 weeks in a dry room (humidity: 20±5%, temperature: 23±4°C) to elicit keratoconjunctival epithelial damage. The eye drop conditions were PBS in one eye and 0.1% selenium-lactoferrin (PBS solution) in the other eye, at 5 µL each time for a total of 4 times per day, for 2 consecutive weeks. For comparison, animals in a normal environment (humidity: 50±5%, temperature: 23±4°C) were also prepared (without eye dropping). Eight animals were used for both the dry room and the normal environment.

Upon completion of 2 weeks of dry room treatment and dropping administration, the corneal epithelial damaged portions were evaluated in the same manner as Example 1, by dyeing with the fluorescent dye fluorescein and scoring. The total score value for the PBS-administered side and the total score value for 0.1% selenium-lactoferrin were compared by a Student's t-test.

### (Results)

The results are shown in Fig. 5. In the graphs, the ordinate represents the average fluorescein score value. The left column of Fig. 5(a) shows the score for a normal environment, and the right column shows the score for dry room treatment (PBS-administered side). The left column of Fig. 5(b) represents the PBS-administered side as a control, and the right column represents the 0.1% selenium-lactoferrin-administered side.

As shown in Fig. 5(a), dry room treatment significantly elicited keratoconjunctival epithelial damage (p <0.05). The keratoconjunctival epithelial damage caused by the dry room was clearly inhibited to a significant degree (p <0.05) by administration of 0.1% selenium-lactoferrin (fluorescein score average: 2.1), compared to the control (PBS-administered side, fluorescein score average: 3.4), as shown in Fig. 5(b).

As clearly seen by the results of the pharmacological test, it was confirmed that eye dropping administration of selenium-lactoferrin inhibited progression of corneal epithelial damage, the effect being greater than with apolactoferrin. Thus, selenium-lactoferrin is useful as a prophylactic or therapeutic agent for corneal and conjunctival diseases such as dry eye, keratoconjunctivitis sicca, superficial punctate keratopathy, corneal erosion and corneal ulcer, and particularly as a prophylactic or therapeutic agent for such diseases accompanied by keratoconjunctival epithelial damage.

### [Formulation Example 1]

A typical formulation example for eye drops containing the selenium-lactoferrin of the invention is shown in Table 1 below. In the Table 1, the concentration of each component is the amount of the component per 100 mL of sterilized distilled water.

**[Table 1]**

| Composition | Concentration (/100 mL sterilized distilled water |
|---|---|
| Glucose | 3 mg |
| Urea | 10 mg |
| Sodium | 12 mEq |
| Potassium | 2.0 mEq |
| Calcium | 0.1 mEq |
| Chlorine | 12 mEq |
| Bicarbonic acid | 2.2 mEq |
| Selenium-lactoferrin | 150 mg |

### Industrial Applicability

The selenium-lactoferrin of the invention has an inhibiting effect on progression of corneal epithelial damage, and is therefore useful for prevention or treatment of corneal and conjunctival diseases such as dry eye, keratoconjunctivitis sicca, superficial punctate keratopathy, corneal erosion and corneal ulcer. In addition, the selenium-lactoferrin of the invention uses lactoferrin as the starting material, lactoferrin being abundantly and readily available, and it can therefore be produced at low cost on a large industrial scale.

In other words, the invention provides a therapeutic agent for corneal and conjunctival diseases that has a high therapeutic effect and high safety and is suitable for large-scale industrial production, and it is therefore industrially useful.

## Claims

1. A metalloprotein that is selenium-lactoferrin.

2. The metalloprotein according to claim 1, wherein selenium is bonded to an iron-binding pocket in apolactoferrin.

3. The process for producing the metalloprotein according to claim 1, comprising a step of adding a selenium salt to a solution containing lactoferrin and/or apolactoferrin, and a step of subjecting the obtained mixed solution to dialysis or ultrafiltration.

4. The process according to claim 3, employing a solution that contains apolactoferrin.

5. The process for producing a metalloprotein according to claim 4, wherein apolactoferrin is produced by a process comprising a step of supplying a solution containing lactoferrin and an acid to an ultrafiltration membrane and conducting ultrafiltration, and a step of recovering the solution that has not permeated the ultrafiltration membrane.

6. A metalloprotein produced by the process according to claim 3.

7. A prophylactic or therapeutic agent for corneal and conjunctival diseases, comprising a metalloprotein according to claim 1 as an active ingredient.

8. The prophylactic or therapeutic agent according to claim 7, wherein the corneal or conjunctival disease is dry eye, keratoconjunctivitis sicca, superficial punctate keratopathy, corneal erosion or corneal ulcer.

9. The prophylactic or therapeutic agent according to claim 7, which is an eye drop or eye ointment.

10. A prophylactic or therapeutic agent for dry eye, keratoconjunctivitis sicca, superficial punctate keratopathy, corneal erosion or corneal ulcer, comprising a metalloprotein according to claim 1 as an active ingredient.

11. An ophthalmologic composition comprising a metalloprotein according to claim 1 and an ophthalmological carrier that is acceptable for ophthalmological formulations.
